(19) Europäisches Patentamt
European Patent Office
Office européen des brevets

(11) Veröffentlichungsnummer: **0 440 319 A1**

(12) # EUROPÄISCHE PATENTANMELDUNG

(21) Anmeldenummer: **91250015.4**

(22) Anmeldetag: **23.01.91**

(51) Int. Cl.[5]: **C07D 513/04**, C07D 207/08, C07D 211/22, C07C 331/28, //C07C335/16,A01N43/90

(30) Priorität: **25.01.90 DE 4002366**

(43) Veröffentlichungstag der Anmeldung: **07.08.91 Patentblatt 91/32**

(84) Benannte Vertragsstaaten: **AT BE CH DE DK ES FR GB GR IT LI LU NL SE**

(71) Anmelder: **SCHERING AKTIENGESELLSCHAFT**
**Müllerstrasse 170/178 Postfach 65 03 11**
**W-1000 Berlin 65(DE)**

(72) Erfinder: **Franke, Wilfried, Dr.**
**Spiessergasse 6b**
**W-1000 Berlin 27(DE)**
Erfinder: **Dorfmeister, Gabriele, Dr.**
**Heiligenseestrasse 70**
**W-1000 Berlin 27(DE)**
Erfinder: **Ganzer, Michael, Dr.**
**Eichborndamm 279**
**W-1000 Berlin 26(DE)**

(54) **Verfahren und Zwischenprodukte zur Herstellung von anellierten Iminothiazolen.**

(57) The invention relates a new process for the the preparation of anellated iminothiazoles of general formula I

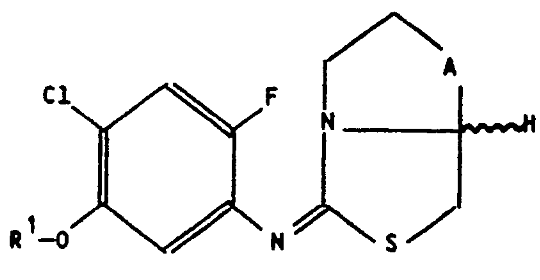

(I) ,

in which A is the group $-CH_2-$ or $-CH_2CH_2-$ and $R^1$ is $C_1$-$C_6$-alkyl, $C_2$-$C_6$-alkenyl, $C_3$-$C_6$-alkynyl or $C_1$-$C_6$-alkoxycarbonyl-$C_1$-$C_6$-alkyl, which starts from the known aniline of general formula II

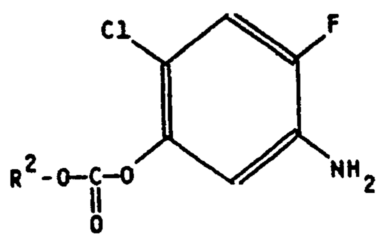

(II) ,

in which $R^2$ is $C_1$-$C_6$-alkyl or phenylmethyl, and goes via new isothiocyanates of general formula IV

EP 0 440 319 A1

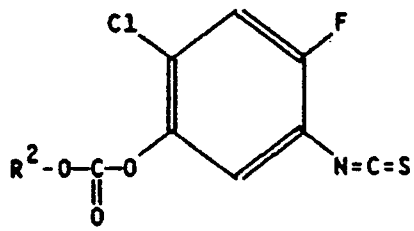

(IV) ,

new thioureas of general formula VI

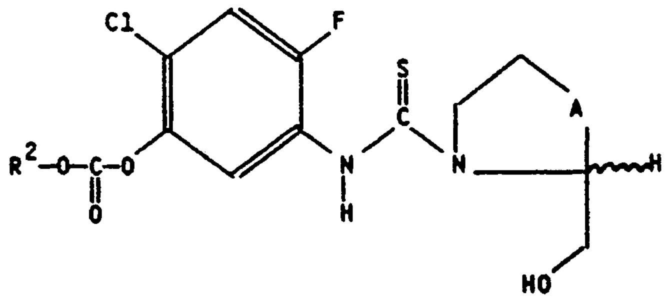

(VI) ,

and new anellated iminothiazole of general formula VII

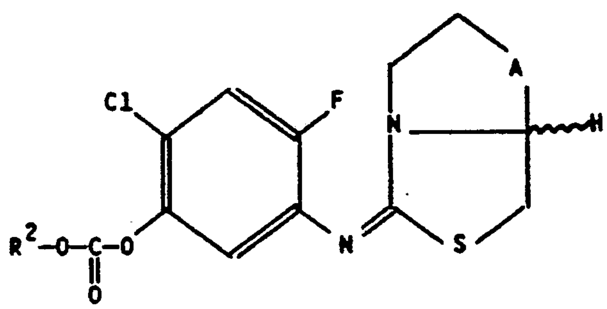

(VII) ,

## PROCESS AND INTERMEDIATES FOR THE PREPARATION OF ANNELATED IMINOTHIAZOLES

This invention relates to a new process and new intermediates for the preparation of anellated iminothiazoles of general formula I

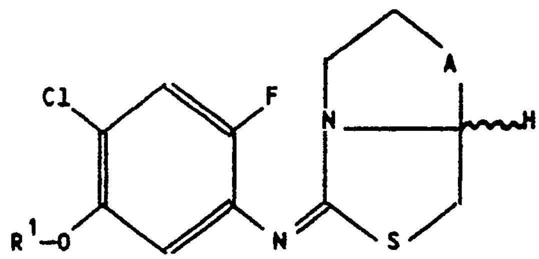

(I) ,

in which A is the group $-CH_2-$ or $-CH_2CH_2-$ and $R^1$ is $C_1$-$C_6$-alkyl, $C_2$-$C_6$-alkenyl, $C_3$-$C_6$-alkynyl or $C_1$-$C_6$-alkoxycarbonyl-$C_1$-$C_6$-alkyl.

These compounds have excellent herbicidal activity against a broad spectrum of monocotyledonous and dicotyledonous weeds in agricultural crops. Their preparation and use are described in EP 298405.

The object of the present invention is to provide a new process which allows a preparation of the compounds of general formula I without any problems, under mild reaction conditions.

This object is solved by a process for the preparation of anellated iminothiazoles in which:

a) an aniline of general formula II

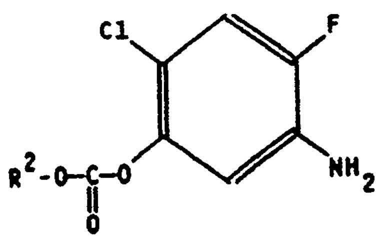

(II) ,

in which $R^2$ is $C_1$-$C_6$-alkyl or phenylmethyl, is reacted with thiophosgene of formula III

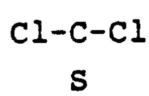

(III)

in the presence of an acid binding agent in a suitable solvent,

b) the isothiocyanate so formed, of general formula IV

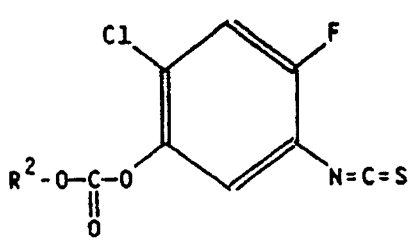

(IV) ,

in which $R^2$ has a meaning given in general formula II is reacted with a cyclic aminoalcohol of general formula V

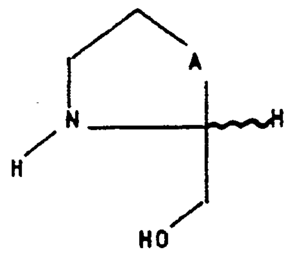

(V) ,

in which A has the meaning given in general formula I, optionally in the presence of catalytic to equimolar amounts of a tertiary amine, in a suitable solvent,

c) the thiourea so formed, of general formula VI

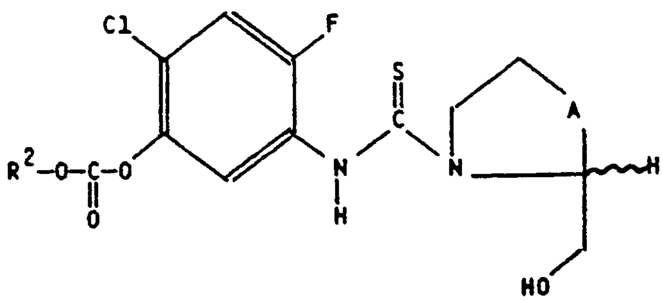

(VI) ,

in which A has the meaning given under general formula I and $R^2$ has the meaning given under general formula II, is cyclised with a mineral acid in a suitable solvent,

d) the anellated iminothiazole so formed, of general formula VII

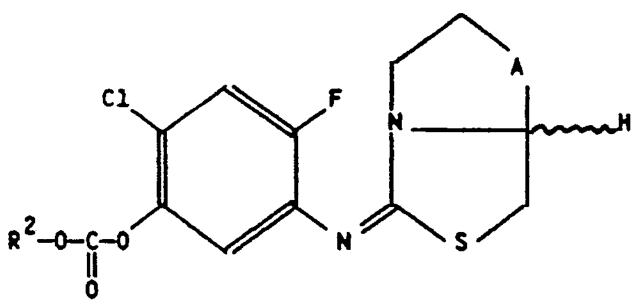

(VII) ,

in which A and $R^2$ have the meanings given in general formula VI, is hydrolysed under basic or acidic conditions, and finally

e) the anellated hydroxyiminothiazole so formed, of general formula VIII

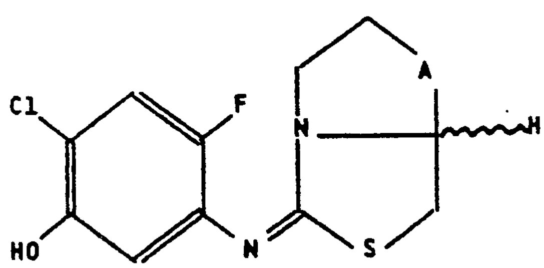

(VIII) ,

in which A has the meaning given in general formula I is reacted with a compound of general formula IX

$R^1$-X (IX)

in which $R^1$ has the meaning given in general formula I and X is chlorine, bromine, iodine, p-toluenesulphonyloxy or methanesulphonyloxy, optionally with the addition of a base in a suitable solvent.

The preparation of the iminothiazole carried out by the new process offers surprising advantages in the state of the art. Instead of having to carry out the cyclisation of the thiourea of general formula VI to the iminothiazole of general formula VIII at high temperatures, as would havre been expected, surprisingly this can be carried out under exceptionally mild conditions at room temperature. The drastic reaction conditions of the original method would also lead to undesired by-products and therby with contaminants which makes purification very difficult or impossible. These problems are completely solved with the new process.

A further unexpected advantage of the new process is that the cleavage of the -CO-O-$R^2$ group to the phenol can be carried out surprisingly under very mild conditions, without the need for high temperatures, and results in better yields. In the original process, the alkoxycarbonyl group was already cleaqveds at a much earlier stage (see EP 61741) and then the $R^1$ group is immediately put on. This again leads to further of the above mentioned problems which however do not occur with the use of the CO-O-$R^2$ group.

A further improvement in the state of the art is that the reaction stages b), c) and d) can be carried out in a so-called one-pot reaction. Surprisingly compounds of general formula VIII are obtained in a high yield and without impurities if in each case, in the resulting reaction, solvent is entirely removed and the so obtained crude product is used without purification in the next stage.

The compounds prepared in the individual reaction stages can optionally be formed in various enantiomeric forms and are also within the scope of this invention.

The anilines of general formula II, which are starting materials for the reaction stage a) can be obtained by reduction of the corresponding nitro compounds in known manner (EP 241559). The synthesis of the corresponding nitro compounds is described in EP 61741.

The isothiocyanate used as starting materials in reaction stage b) of general formula IV are new and are also within the scope of the invention. They can be prepared from the anilines of general formula II by known methods in high yields. It has been shown that the reaction with thiophosgene can be carried out suitably in the presence of calcium carbonate in a two phase system of ethyl acetate and water.

The thioureas used as starting materials for reaction stage c) of general formula VI are also new and are therefore also within the scope of this invention. They can optionally be present in various diastereomeric and enantiomeric forms and these are also within the scope of this invention. They can be prepared from the isothiocyanate of general formula IV by reaction with the cyclic aminoalcohols of general formula V. The reaction can optionally be carried out in a suitable solvent with the addition of catalytic to equimolar amounts of a tertiary amine, such as for example triethylamine or pyridine.

Suitable solvents include hydrocarbons, such as for example toluene, chlorinated hydrocarbons, such as for example methylene chloride or chloroform, ethers, such as for example diethyl ether, dioxane or tetrahydrofuran, alcohols, such as for example methanol or ethanol, ketones, such as for example acetone or butanone, amides, such as for example dimethylformamide and also sulphoxides.

The reaction time is usually between 0.5 to 15 hours, with a reaction temperature in the range of 20°C up to the boiling point of a particular solvent.

The starting material for the reaction stage d) to the anellated iminothiazole of general formula VII are new and are within the scope of the invention. They can be prepared by cyclising thioureas of general formula VI with a mineral acid for example hydrochloric acid, hydrobromic acid or sulphuric acid, in a suitable solvent at a temperature of 20 to 100°C, preferably however at room temperature.

Examples of solvents are especially ethers, such as diethyl ether, tetrahydrofuran or dioxane, or alcohols, such as methanol or ethanol.

The compounds of general formula VIII, used as staring materials in reaction stage e), can be prepared in high yields under mild conditions by hydrolysing compounds of general formula VII under basic or acidic conditions. When $R^2$ = phenylmethyl, it is also possible to obtain the compounds of general formula VII by a hydrogenolysis.

In the case of a basic hydrolysis, the starting material is suitably hydrolysed with an aqueous or aqueous-alcoholic solution of an alkaline metal or alkaline earth metal hydroxide, alkali metal or alkaline earth metal carbonate or hydrogen carbonate, at temperatures of 20°C to 150°C, preferably at room temperature, to give the corresponding derivative.

Acid hydrolysis is preferably carried out in a suitable solvent with a mineral or carboxylic acid in a temperature range of 20 to 150°C. Suitable solvents include hydrocarbons, such as for example toluene, chlorinated hydrocarbons, such as for example methylene chloride or chloroform, ethers such as for example diethyl ether or tetrahydrofuran, alcohols such as for example methanol or ethanol, or an aqueous alcoholic solvent. Examples of mineral and carboxylic acids, include hydrochloric acid, sulphuric acid, p-toluenesulphonic acid and trifluoroacetic acid.

The hydrogenolysis can be carried out under the usual conditions for a catalytic hydrogenation.

The compounds of general formula VIII are then treated with compounds of general formula IX to give the anellated iminothiazoles of general formula I.

The process is suitably carried out by reacting the starting materials in a suitable solvent, optionally with the addition of an inorganic or organic base, at temperatures between 0° C and 150° C, preferably at the reflux temperature of the solvent.

The reaction can also be carried out by the addition of a phased transfer catalyst.

Suitable bases include alkali metal and alkaline earth metal hydroxides, alcoholates, alkali metal hydrides, alkali metal and alkaline earth metal carbonates and hydrogen carbonates, tertiary aliphatic and aromatic amines as well as heterocyclic bases. Examples are sodium and potassium hydroxide, sodium methanolate, sodium hydride, sodium and potassium carbonate, sodium and potassium hydrogen carbonate, triethylamine and pyridine. Suitable solvents include hydrocarbons, such as for example toluene, chlorinated hydrocarbons, such as for example methylene chloride or chloroform, ethers, such as for example diethyl ether or tetrahydrofuran, alcohols, such as for example methanol or ethanol, ketones, such as for example acetone or butanone, amides, such as for example dimethylformamide, sulphoxides, such as for example dimethyl sulphoxide, and also nitriles, such as for example acetonitrile.

The invention is illustrated in the following examples.

Example 1 - Reaction stage a)

4-Chloro-2-fluoro-5-methoxycarbonyloxyphenyl isothiocyanate

A solution of 117,7 g 4-chloro-2-fluoro-5-(methoxy-carbonyloxy)aniline in 500 ml methyl acetate was added dropwise to a two phase mixture comprising 61.63 g thiophosgene in 200 ml ethyl acetate and 53.6 g calcium carbonate in 200 ml water at a temperature of 5-10° C. After an hour, the organic phase was separated, dried over magnesium sulphate and after filtering, the solvent was removed *in vacuo* and the residue dried.

Yield: 137.2 g = 98% of theory
mp: 75° C

In similar manner the compounds of formula IV as shown in the following table, were obtained.

| $R^2$ | A | Physical Constant |
|---|---|---|
| $-C_2H_5$ | $-CH_2-$ | mp: 46°C |
| $-CH(CH_3)_2$ | $-CH_2-$ | mp: 27°C |
| $-CH_2C_6H_5$ | $-CH_2-$ | mp: 70°C |

The starting materials of general formula II used in example 1 were prepared in known manner (EP 241 559). Compounds of general formula II in which $R^2$ = $-CH(CH_3)_2$ (mp: 112° C) and $R^2$ = $-CH_2C_6H_5$ (mp: 67° C) are new and not known in the literature, up until now.

Example 2 - Reaction stage b)

(s)-(-)-1-[(4-Chloro-2-fluoro-5-methoxycarbonyloxyphenyl)thiocarbamoyl]-2-(hydroxymethyl)pyrrolidine

2,02 g (S)-(+)-2-(Hydroxymethyl)pyrrolidine in 20 ml tetrahydrofuran was treated under ice-cooling with a.solution of 5,24 g 4-chloro-2-fluoro-5-methoxy-carbonyloxyphenyl isothiocyanate in 30 ml tetrahydrofuran and the mixture stirred at room temperature. After the reaction had finished, the solvent was removed *in vacuo* and the residue recrystallised from isopropyl ether and a small amount of methanol.

Yield: 5.41 g = 75% of theory
mp: 156° C
$[\alpha]_D$: -170.4°

In a similar manner, the compounds of general formula VI as shown in the following table, were

obtained.

| $R^2$ | A | Config. | $[\alpha]_D$ | Physical Constant |
|---|---|---|---|---|
| $-C_2H_5$ | $-CH_2-$ | S | -168.1° | mp: 139°C |
| $-CH(CH_3)_2$ | $-CH_2-$ | S | -159.4° | mp: 107°C |
| $-CH_2C_6H_5$ | $-CH_2-$ | S | -117.8° | glass |
| $-CH_3$ | $-CH_2-$ | R | -169.8° | mp: 154°C |
| $-C_2H_5$ | $-CH_2-$ | R | +168.0° | mp: 139°C |
| $-CH(CH_3)_2$ | $-CH_2-$ | R | +159.9° | mp: 108°C |
| $-CH_2C_6H_5$ | $-CH_2-$ | R | +118.0° | glass |
| $-CH_3$ | $-(CH_2)_2-$ | racemic | - | mp: 50°C |

<u>Example 3</u> - Reaction stage c)

(S)-(-)-N-(4-Chloro-2-fluoro-5-methoxycarbonyloxyphenyl)perhydropyrrolo[1,2-c]thiazol-3-imine

8,73 g (S)-(-)-1-[(4-Chloro-2-fluoro-5-methoxycarbonyloxyphenyl)thiocarbamoyl]-2-(hydroxymethyl)-pyrrolidine was dissolved in 100 ml of ethanol, saturated with hydrogen chloride and the mixture stirred at room temperature. After the reaction had finished, the ethanol was distilled, the residue dissolved in water, treated with ethyl acetate and neutralised with sodium carbonate. The organic phase was concentrated and purified by silica gel chromatography (eluent: hexane/ethyl acetate).

Yield: 6.3 g = 76% of theory

$n_D^{20}$: 1.5848

$[\alpha]_D$: -47.8°

In a similar manner, the compounds of formula VII shown in the following table, were obtained.

| $R^2$ | A | Config. | $[\alpha]_D$ | Physical Constant |
|---|---|---|---|---|
| $-C_2H_5$ | $-CH_2-$ | S | -42.3° | $n_D^{20}$: 1.5771 |
| $-CH(CH_3)_2$ | $-CH_2-$ | S | -42.5° | $n_D^{20}$: 1.5619 |
| $-CH_2C_6H_5$ | $-CH_2-$ | S | -44.1° | $n_D^{20}$: 1.6041 |
| $-CH_3$ | $-CH_2-$ | R | +49.0° | $n_D^{20}$: 1.5850 |
| $-C_2H_5$ | $-CH_2-$ | R | +42.1° | $n_D^{20}$: 1.5769 |
| $-CH(CH_3)_2$ | $-CH_2-$ | R | +42.6° | $n_D^{20}$: 1.5622 |
| $-CH_2C_6H_5$ | $-CH_2-$ | R | +43.9° | $n_D^{20}$: 1.6043 |
| $-CH_3$ | $-(CH_2)_2-$ | racemic | - | $n_D^{20}$: 1.5829 |

Example 4 - Reaction stage d)

(S)-(-)-N-(4-Chloro-2-fluoro-5-hydroxyphenyl)perhydropyrrolo[1,2-c]thiazol-3-imine

1.46 g (S)-(-)-N-[(4-Chloro-2-fluoro-5-methoxycarbonyloxyphenyl)perhydropyrrolo[1,2-c]thiazol-3-imine was stirred at 40° C for three hours in 40 ml 2N caustic soda and 40 ml ethanol and then concentrated to dryness in vacuo. The residue was dissolved in water, acidified with hydrcchloric acid, the precipitate filtered off, washed with water and isopropyl ether and dried.

Yield: 1.20 g = 99% of theory
mp: 159° C
$[\alpha]_D$: -129.2°

In a similar manner the compounds of general formula VIII, shown in the following table, were prepared:

| A | Configuration | $[\alpha]_D$ | Physical Constant |
|---|---|---|---|
| $-CH_2-$ | R | +130.0° | mp: 158°C |
| $-(CH_2)_2-$ | racemic | - | mp: 140°C |

Compounds of general formula VIII can also be obtained in a one pot reaction from the isothiocyanate of formula IV. Example 5 describes this process.

Example 5 - Reaction stage b), c) and d)

(S)-(-)-N-(4-Chloro-2-fluoro-5-hydroxyphenyl)perhydropyrrolo[1,2-c]thiazol-3-imine

A solution of 5.24 g 4-chloro-2-fluoro-5-methoxycarbonyloxyphenyl isothiocyanate in 20 ml tetrahydrofuran was added dropwise to a solution of 2.02 g (S)-(+)-2-(hydroxymethyl)pyrrolidine in 20 ml tetrahydrofuran at 20° C. After an hour, the tetrahydrofuran was removed, the residue was taken up in 75 ml of ethanol saturated with hydrogen chloride and the mixture stirred for 12 hours at room temperature. The solvent was removed, the residue stirred for 3 hours in 150 ml 2 n caustic soda at 40° C and then concentrated to dryness in vacuo. The residue was dissolved in water, acidified with hydrochloric acid, the precipitate separated, washed with water and isopropyl ether and dried.

Yield: 4.9 g = 85% of theory
mp: 159° C
$[\alpha]_D$: -129.2°

In a similar manner, the following compounds of general formula III were obtained.

| A | Configuration | $[\alpha]_D$ | Physical Constant |
|---|---|---|---|
| $-CH_2-$ | R | +130.0° | mp: 158°C |
| $-(CH_2)_2-$ | racemic | - | mp: 140°C |

Example 6 - Reaction stage e)

(S)-(-)-N-[4-Chloro-2-fluoro-5-(2-propynyloxyphenyl)-perhydropyrrolo[1,2-c]thiazol-3-imine

A mixture of 1.97 g (S)-(-)-N-(4-chloro-2-fluoro-5-hydroxyphenyl)perhydropyrrolo[1,2-c]thiadiazol-3-imine, 0.95 g calcium carbonate and 1.03 g 3-chloro-1-propyne in 50 ml acetone was heated at boiling point for 20

hours. The precipitate was filtered, the acetone distilled and the residue washed with hexane and dried *in vacuo*.

Yield: 2.04 g = 91% of theory
mp: 113 °C
$[\alpha]_D$: -61.0°

In a similar manner, the compounds of general formula I, shown in the following table, were prepared:

| $R^2$ | A | Config. | $[\alpha]_D$ | Physical Constant |
|---|---|---|---|---|
| $-CH_2C{\equiv}CH$ | $-CH_2-$ | R | +60.9° | mp: 113°C |
| $-CH_3$ | $-CH_2-$ | S | -64.0° | mp: 113°C |
| $-CH(CH_3)_2$ | $-CH_2-$ | S | -59.6° | mp: 78°C |
| $-CH_2CO_2C_2H_5$ | $-CH_2-$ | S | -57.6° | mp: 67°C |
| $-CH_3$ | $-CH_2-$ | R | +61.9° | mp: 115°C |
| $-CH(CH_3)_2$ | $-CH_2-$ | R | +60.3° | mp: 81°C |
| $-CH_2CO_2C_{25}$ | $-CH_2-$ | R | +127.8° | mp: 67°C |
| $-CH_3$ | $-(CH_2)_2-$ | racemic | - | mp: 76°C |
| $-CH(CH_3){\equiv}CH$ | $-CH_2-$ | RS;SS | -27.0° | $n_{D20}$: 1.5932 |

## Claims

1. A process for the preparation of anellated iminothiazoles of general formula I

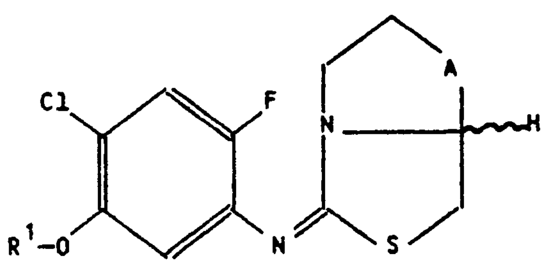

(I) ,

in which A is the group $-CH_2-$ or $-CH_2CN_2-$ and $R^1$ is $C_1$-$C_6$-alkyl, $C_2$-$C_6$-alkenyl, $C_3$-$C_6$-alkynyl or $C_1$-$C_6$-alkoxycarbonyl-$C_1$-$C_6$-alkyl, in which:

a) an aniline of general formula II

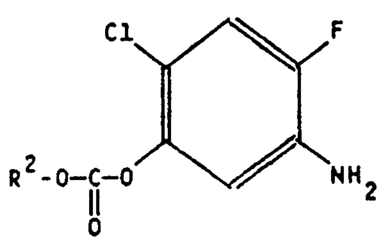

(II) ,

in which $R^2$ is $C_1$-$C_6$-alkyl or phenylmethyl, is reacted with thiophosgene of formula III

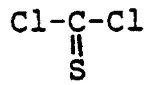

(III)

in the presence of an acid binding agent in a suitable solvent,
b) the isothiocyanate so formed, of general formula IV

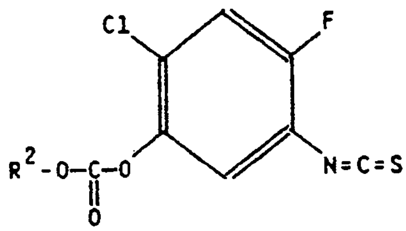

(IV) ,

in which $R^2$ has a meaning given in general formula II is reacted with a cyclic aminoalcohol of general formula V

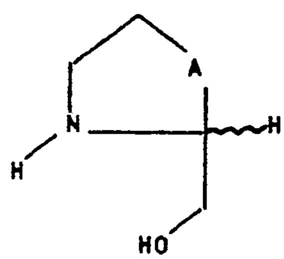

(V) ,

in which A has the meaning given in general formula I, optionally in the presence of catalytic to equimolar amounts of a tertiary amine, in a suitable solvent,
c) the thiourea so formed, of general formula VI

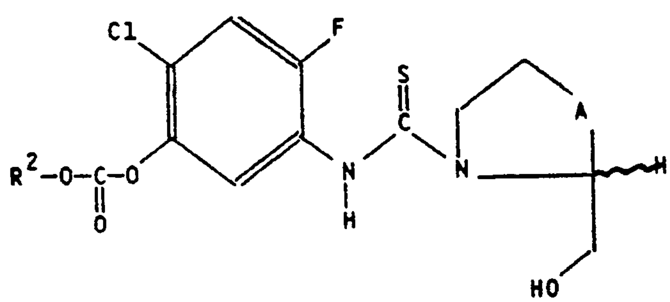

(VI) ,

in which A has the meaning given under general formula I and $R^2$ has the meaning given under general formula II, is cyclised with a mineral acid in a suitable solvent,
d) the anellated iminothiazole so formed, of general formula VII

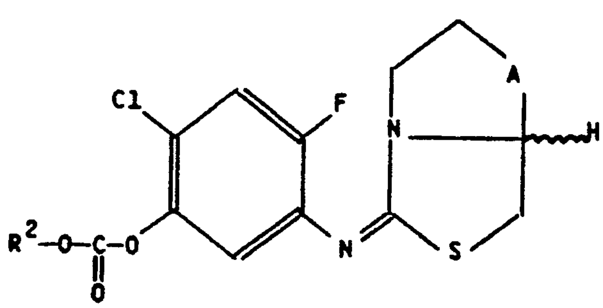

(VII) ,

in which A and $R^2$ have the meanings given in general formula VI, is hydrolysed under basic or acidic conditions, and finally

e) the anellated hydroxyiminothiazole so formed, of general formula VIII

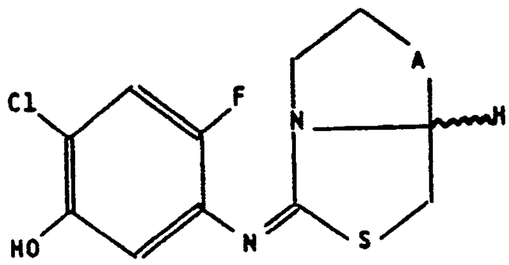

(VIII) ,

in which A has the meaning given in general formula I is reacted with a compound of general formula IX

$R^1$-X (IX)

in which $R^1$ has the meaning given in general formula I and X is chlorine, bromine, iodine, p-toluenesulphonyloxy or methanesulphonyloxy, optionally with the addition of a base in a suitable solvent.

2. A process according to claim 1, in which the reaction stages b), c) and d) are carried out in a so-called one-pot reaction, in which after each individual reaction stage, solvent is entirely removed and the so obtained crude product is used without purification in the next stage.

3. Isothiocyanates of general formula IV

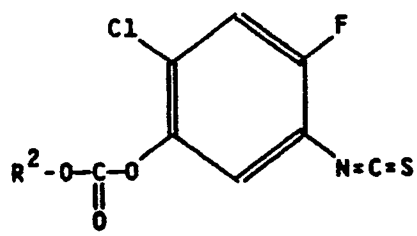

(IV) ,

in which $R^2$ is $C_1$-$C_6$-alkyl or phenylmethyl.

4. Thioureas of general formula VI

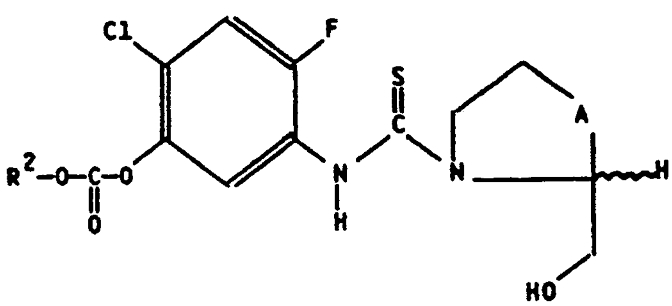

(VI) ,

in which A is the group $-CH_2-$ or $-CH_2CH_2-$ and $R^2$ is $C_1$-$C_6$-alkyl or phenylmethyl.

5. Anellated iminothiazoles of general formula VII

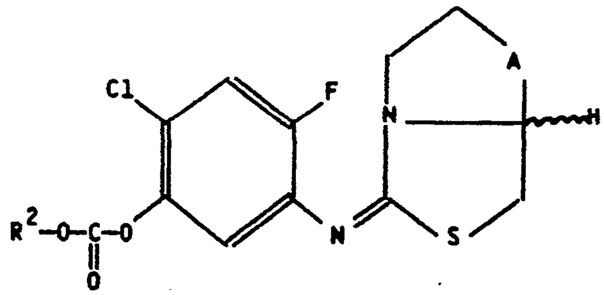

(VII) ,

in which A is the group $-CH_2-$ or $-CH_2CH_2-$ and $R^2$ is $C_1$-$C_6$-alkyl or phenylmethyl.

Europäisches Patentamt

# EUROPÄISCHER RECHERCHENBERICHT

Nummer der Anmeldung

EP 91250015.4

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl 5) |
|---|---|---|---|
| D,X | EP - A2 - 0 298 405 (SCHERING AKTIENGESELLSCHAFT) * Claims * | 1,3-5 | C 07 D 513/04<br>C 07 D 207/08<br>C 07 D 211/22<br>C 07 C 331/28<br>//C 07 C 335/16<br>A 01 N 43/90 |
| A | CHEMICAL ABSTRACTS, vol. 109, no. 1, July 4, 1988, Columbus, Ohio, USA K. HAGIWARA et al. "Preparation of thiadiazole analogs as selective herbicides." page 620, abstract-no. 6 525r & Jpn. Kokai Tokkyo Koho JP 62,181,283 (87,181,283) | 1,3 | |
| A | DE - A1 - 3 528 583 (NIPPON SODA CO. LTD.) * Claims 5-7 * | 1,3 | |
| A | EP - B1 - 0 075 267 (SUMITOMO CHEMICAL COMPANY LIMITED) * Claims 9-12,17 * | 3,4 | **RECHERCHIERTE SACHGEBIETE (Int Cl 5)** |
| A | DE - A1 - 3 643 748 (HOECHST AG) * Claim 3; table I * | 3 | C 07 D<br>C 07 D 331/00<br>A 01 N |
| A | EP - A2 - 0 290 902 (BAYER AG) * Example (II-2), table 2; claim 10 * | 3 | |
| A | DE - A1 - 3 601 048 (HOECHST AG) * Table 1 * | 3,4 | |

Der vorliegende Recherchenbericht wurde für alle Patentansprüche erstellt

| Recherchenort | Abschlußdatum der Recherche | Prüfer |
|---|---|---|
| WIEN | 06-05-1991 | SCHNASS |

KATEGORIE DER GENANNTEN DOKUMENTEN

X von besonderer Bedeutung allein betrachtet
Y von besonderer Bedeutung in Verbindung mit einer anderen Veröffentlichung derselben Kategorie
A technologischer Hintergrund
O nichtschriftliche Offenbarung
P Zwischenliteratur
T der Erfindung zugrunde liegende Theorien oder Grundsätze

E älteres Patentdokument, das jedoch erst am oder nach dem Anmeldedatum veröffentlicht worden ist
D in der Anmeldung angeführtes Dokument
L aus andern Gründen angeführtes Dokument

& Mitglied der gleichen Patentfamilie, übereinstimmendes Dokument